Europäisches Patentamt

European Patent Office

Office européen des brevets

(11) Numéro de publication: **0 688 864 A1**

# DEMANDE DE BREVET EUROPEEN

(21) Numéro de dépôt: **94109539.0**

(22) Date de dépôt: **21.06.94**

(51) Int. Cl.6: **C12N 1/04**, A23C 9/123

(43) Date de publication de la demande:
**27.12.95 Bulletin 95/52**

(84) Etats contractants désignés:
**AT BE CH DE DK ES FR GB GR IE IT LI LU NL PT SE**

(71) Demandeur: **SOCIETE DES PRODUITS NESTLE S.A.**
**Case postale 353**
**CH-1800 Vevey (CH)**

(72) Inventeur: **Mignot, Olivier**
**5, rue des Perdrix**
**F-95800 Courdimanche (FR)**

(74) Mandataire: **Wavre, Claude-Alain et al**
**55, avenue Nestlé**
**CH-1800 Vevey (CH)**

(54) **Starter congelé de yogourt**

㊄ Procédé de préparation d'un starter congelé pour l'inoculation directe d'un lait dans le but d'en faire du yogourt, dans lequel on cultive des bactéries lactiques dans du lait à une température de 40-44 °C jusqu'à un pH de 4,3-5,5, puis l'on congèle directement la culture. L'invention concerne également le starter congelé et l'utilisation de ce starter.

EP 0 688 864 A1

Rank Xerox (UK) Business Services
(3.10/3.09/3.3.4)

La présente invention a pour objet un procédé de préparation d'un starter congelé pour l'inoculation directe d'un lait et le starter congelé obtenu par ce procédé.

Il est connu de conserver une culture de bactéries lactiques par congélation en vue de l'utiliser pour la fabrication de yogourt. Une telle culture, appelée encore starter, peut être soit réactivée par préculture avant d'être inoculée à un lait, ou soit être inoculée directement, sous forme congelée, à un lait pour en faire du yogourt.

US 3975545 décrit ainsi la préparation d'un starter congelé de yogourt réactivé avant d'être inoculé à un lait. Dans ce procédé, on cultive des bactéries lactiques dans un milieu synthétique, on les concentre, on leur ajoute un cryoprotectant, à savoir l'acide glycérophosphorique, puis on les congèle à -20°C. Ce starter est ensuite réactivé par préculture dans un milieu synthétique ou dans du lait comprenant le cryoprotectant, puis est inoculé à un lait pour en faire du yogourt.

De même, EP 0378478 décrit la préparation d'un starter congelé pour l'inoculation directe d'un lait. Dans ce procédé, on cultive des bactéries lactiques dans un milieu de nutrition synthétique qui comprend notamment des sels de polyphosphate pour solubiliser la caséine, on concentre la culture, puis on la congèle. Un tel starter comprend, une fois congelé, environ $10^9$ à $10^{10}$ colonies de cellules vivantes par gramme ou cfu/g (cfu est l'abréviation anglaise de "colony forming unit"), et est utilisé classiquement en inoculation directe à raison de 0,01-0,1%.

Les bactéries lactiques présentes dans un starter congelé utilisé pour l'inoculation directe ont cependant été soumises à des stress qui influent sur leur capacité à fermenter normalement du lait.

Ces stress peuvent être dus, par exemple, à la culture des bactéries dans un milieu de nutrition synthétique dont la composition est plus ou moins éloignée de celle du lait. Les bactéries développent alors un métabolisme qui est peu adapté à une fermentation du lait. De même, les bactéries sont concentrées, par exemple par centrifugation, et sont de ce fait également perturbées. Enfin, on ajoute classiquement à la culture, avant congélation, un cryoprotectant de manière à protéger les cellules de la congélation. Cependant, ces cryoprotectants peuvent également avoir un effet inhibiteur sur la croissance des bactéries.

Les bactéries vivantes d'un tel starter étant ainsi perturbées, seulement une fraction d'entre elles sont réellement capables de fermenter du lait et donc de produire de l'acide lactique. On a pu ainsi déterminer que moins de 10% de ces bactéries inoculées fermentent effectivement le lait. Il serait donc très intéressant d'augmenter le taux d'activité fermentaire d'un starter congelé.

La présente invention a pour but de pallier les inconvénients de l'art antérieur, et de proposer ainsi un starter congelé pour l'inoculation directe d'un lait présentant un taux d'activité fermentaire supérieur à 10%, et de préférence supérieur à 95%.

A cet effet, dans le procédé de préparation d'un starter congelé pour l'inoculation directe d'un lait en vue d'en faire du yogourt, on cultive des bactéries lactiques dans du lait à une température de 40-45°C jusqu'à un pH de 4,3-5,5, puis on congèle directement la culture.

Le starter congelé et son utilisation pour la fabrication de yogourt sont également l'objet de l'invention.

Ainsi, le procédé de préparation d'un starter congelé selon la présente invention présente l'avantage de ne pas faire intervenir un milieu de culture synthétique et un dispositif de concentration. Ce procédé est donc particulièrement peu onéreux, et simple à mettre en oeuvre.

De plus, on a constaté avec surprise que le starter congelé, selon la présente invention, présente un taux d'activité fermentaire supérieur à 95%, ce qui signifie que parmi les bactéries vivantes ayant survécu à la congélation, plus de 95% des bactéries sont capables de fermenter rapidement le lait, et par conséquent de produire de l'acide lactique.

Ainsi, le starter objet de la présente invention peut fermenter un lait jusqu'à un pH traditionnel de yogourt, c'est à dire de l'ordre de 4,4 à 4,8, aussi rapidement que peut le faire un starter congelé traditionnel pour l'inoculation directe, alors que ce dernier contient au moins 10 fois plus de cellules vivantes.

Enfin, ce procédé peut être mis en oeuvre sans utiliser un cryoprotectant, et cela particulièrement pour la préparation d'un starter congelé de streptocoques.

Dans la suite de la description, le terme "streptocoque" comprend les lactocoques et les streptocoques selon la nouvelle nomenclature (Bergey's Manual).

Pour mettre en oeuvre le présent procédé, on peut donc cultiver dans du lait au moins une souche de streptocoques et/ou au moins une souche de lactobacilles, par exemple. On utilise ainsi, de préférence parmi les streptocoques, *Lactococcus lactis subsp. lactis*, *Lactococcus lactis subsp. cremoris*, *Lactococcus lactis subsp. lactic biovar diacetylactis*, et *Streptococcus thermophilus*. De même, parmi les lactobacilles, on peut utiliser *Lactobacillus delbrueckii subsp. bulgaricus*, *Lactobacillus acidophilus*, *Lactobacillus helveticus*, *Lactobacillus casei subsp. casei*, et *Lactobacillus delbruckii subsp lactis*, par exemple (Bergey's Manual of Systematic Bacteriology, vol 2, 1986).

2

En particulier, pour préparer le starter selon la présente invention, on peut inoculer le lait avec une préculture fraîche de chaque genre ou espèce bactérien utilisé et/ou avec une préculture fraîche comprenant un mélange des genres et espèces bactériens utilisés. Le milieu de préculture peut être, par ailleurs, un milieu de nutrition synthétique habituellement utilisé pour la culture en laboratoire de bactéries lactiques, par exemple le milieu MRS (De Man et al, 1960), ou un milieu à base de lait, par exemple le milieu MSK comprenant du lait écrémé en poudre reconstitué à 10% et 1/10000 parties d'un extrait de levure commerciale. On envisage donc toutes les possibilités d'inoculation avec une ou plusieurs précultures fraîches, comprenant chacune une ou plusieurs souches de bactéries lactiques pouvant être d'un genre ou d'une espèce différente. L'inoculum de chaque préculture fraîche est ainsi ajouté en une quantité qui dépend de la durée nécessaire à la fermentation pour atteindre le pH final, et de la ou les souches de bactéries lactiques utilisées. Enfin, de préférence, on prépare la préculture fraîche en réalisant plusieurs précultures successives des souches initialement congelées ou lyophilisées, et on inocule le lait avec la dernière préculture, généralement la troisième, prise à partir du stade de coagulation du milieu de préculture.

D'autres formes d'inoculation directe, par exemple par des cultures lyophilisées, peuvent être également utilisées.

De préférence, on ajoute à une culture comprenant notamment des lactobacilles, 3-10% de glycérine avant de la congeler. Une telle culture peut ainsi comprendre, en plus des lactobacilles, un autre genre ou espèce bactérien, par exemple une ou plusieurs souches de streptocoques. L'ajout de glycérine permet d'augmenter la survie de ces lactobacilles lors de la congélation, et ainsi d'obtenir un starter selon la présente invention présentant un taux d'activité fermentaire supérieur à 95%. Cependant, il faut remarquer qu'un tel ajout n'est pas une condition obligée pour obtenir un starter selon la présente invention présentant un taux d'activité fermentaire supérieur à 10%.

De même, on peut congeler les bactéries lactiques, d'une manière traditionnelle, entre -20 et -90°C, par exemple.

Enfin, le lait peut être soit un lait écrémé stérile, soit un lait écrémé reconstitué stérile, c'est à dire reconstitué à partir de poudre de lait et stérilisé à 110-120°C pendant 20-40 min, par exemple. Ce lait peut comprendre en outre 1% d'extrait de levure, de manière à améliorer la croissance des bactéries lors de la préparation du starter.

Dans un premier mode de réalisation préféré du procédé selon la présente invention, pour obtenir un starter de streptocoques ayant un taux d'activité fermentaire maximal, on cultive des streptocoques à une température de 41-44°C jusqu'à un pH de 4,9 à 5,2 de manière à obtenir une concentration de cellules de l'ordre de $2.10^8$ à $5.10^8$ cfu/g, puis on congèle directement la culture.

On peut donc cultiver dans le lait plusieurs espèces ou souches de streptocoques. L'inoculum de streptocoques peut être également une préculture fraîche comprenant, en mélange, les espèces ou souches de streptocoques utilisées et/ou plusieurs précultures fraîches de chaque espèce ou souche de streptocoque utilisée. En particulier, on cultive de cette manière au moins une souche de *Streptococcus thermophilus*.

Dans un deuxième mode de réalisation préféré du procédé selon la présente invention, pour obtenir un starter de lactobacilles ayant un taux d'activité fermentaire maximal, on cultive des lactobacilles à une température de 41-44°C jusqu'à un pH de 4,6 à 5 de manière à obtenir une concentration de cellules de l'ordre de $10^8$ à $5.10^8$ cfu/g, puis on congèle directement la culture.

On peut donc cultiver dans le lait plusieurs espèces ou souches de lactobacilles. L'inoculum de lactobacilles peut être également une préculture fraîche comprenant en mélange les espèces ou souches de lactobacilles utilisées et/ou plusieurs précultures fraîches de chaque espèce ou souche de lactobacille utilisée. En particulier, on cultive de cette manière au moins une souche de *Lactobacillus delbruckii subsp. bulgaricus*.

Dans un troisième mode de réalisation préféré du procédé selon la présente invention, pour obtenir un starter mixte comprenant un mélange de lactobacilles et de streptocoques ayant un taux d'activité fermentaire maximal, on cultive lesdites bactéries à une température de 41-44°C jusqu'à un pH de 4,5 à 4,9 de manière à obtenir une concentration de cellules de l'ordre de $3.10^8$ à $5.10^8$ cfu/g, puis on congèle directement la culture.

L'inoculum peut être également une préculture fraîche comprenant en mélange les genres, espèces ou souches bactériens utilisés et/ou plusieurs précultures fraîches de chaque genre, espèce ou souche bactérien utilisé. En particulier, le mélange bactérien cultivé peut comprendre 50-90% de streptocoques par rapport aux lactobacilles, par exemple.

Le starter congelé est également un objet de la présente invention. En effet, on constate avec surprise que plus de 95% des cellules ayant survécu à la congélation sont capables de produire de l'acide lactique.

De même, l'utilisation du starter congelé pour la fabrication de yogourt est l'objet de la présente invention. En effet, un tel starter fermente un lait aussi rapidement que peut le faire un starter traditionnel, alors que ce dernier contient au moins 10 fois plus de cellules vivantes. On préfère ainsi utiliser le starter objet de la présente invention, à raison de 0,01à 0,1% dans du lait.

Le procédé, et le starter congelé selon la présente invention sont illustrés plus en détails dans les exemples présentés ci-après à titre d'illustration. Les pourcentages y sont donnés en poids. Ces exemples sont précédés d'une brève description des dessins.

**Dessins**

Figure 1: fabrication d'un yogourt étuvé. Représentation du pH de deux laits fermentés par des coktails de starters congelés différents, en fonction du temps de fermentation.
Figure 2: fabrication d'un yogourt brassé. Représentation du pH de deux laits fermentés par des coktails de starters congelés différents, en fonction du temps de fermentation.

**Exemple 1**

On prépare un starter congelé d'une souche *Streptococcus thermophilus* commerciale de yogourt, de la manière suivante.

On reconstitue un lait écrémé à partir de 10% de poudre de lait écrémé, on lui ajoute 1% d'extrait de levure, on le stérilise à 115°C pendant 30 min, puis on le refroidit jusqu'à une température d'environ 42°C.

On réactive une souche traditionnelle *S. thermophilus* congelée, par plusieurs précultures successives dans un milieu MSK stérilisé à 155°C pendant 30 min. On inocule ensuite le lait reconstitué stérile à raison de 1% de la troisième préculture prise au stade de coagulation du milieu et on l'incube à une température d'environ 42°C jusqu'à un pH de 5,1, puis on le refroidit jusqu'à une température de 4°C. Finalement, on le congèle à -75°C.

**Exemple 2**

On prépare un starter congelé d'une souche *Lactobacillus delbruckii subsp. bulgaricus* commerciale de yogourt filante, de la manière suivante.

On utilise le même lait écrémé et stérilisé décrit dans l'exemple 1, comprenant 1% d'extrait de levure. On réactive une souche commerciale *L.delbruckii subsp. bulgaricus* congelée, par plusieurs précultures successives dans le milieu MSK stérile. On inocule ensuite le lait reconstitué stérile à raison de 5% de la troisième préculture prise au stade de coagulation du milieu et on l'incube à une température d'environ 42°C jusqu'à un pH de 4,8, puis on le refroidit jusqu'à une température de 4°C. On lui ajoute alors 5% de glycérine stérile et on le congèle à -75°C.

**Exemple 3**

On prépare un starter congelé mixte comprenant un mélange de deux souches commerciales de *Streptococcus thermophilus* et une souche traditionnelle de *Lactobacillus delbruckii subsp. bulgaricus* filante, de la manière suivante.

On prépare d'abord le même lait écrémé et stérilisé décrit dans l'exemple 1, comprenant également 1% d'extrait de levure.

On ajoute à ce lait 1% d'une préculture fraîche de chaque souche de *S. thermophilus* préparées comme décrit à l'exemple 1, et 2% d'une préculture fraîche de la souche *L. delbruckii subsp. bulgaricus* préparée comme décrit à l'exemple 2. On incube ensuite le lait à une température d'environ 42°C jusqu'à un pH de 4,7. On refroidit le lait jusqu'à une température de 4°C. Puis on lui ajoute 5% de glycérine stérile et on le congèle à -75°C.

**Exemple 4**

On prépare des yogourts étuvés par inoculation directe avec des starters congelés tels que décrit dans les exemples 1 et 2.

Un lait est préparé à base de lait entier comprenant 3,7% de matière grasse et 2,5% de poudre de lait écrémé. On pasteurise 40 l de ce lait à 92°C pendant 6 min, on l'homogénéise ensuite à 75°C et 150 bar (deux étages), enfin on le refroidit jusqu'à une température d'environ 42°C.

On réalise alors une fermentation de ce lait avec un cocktail de trois starters congelés comprenant respectivement une souche *S. thermophilus* CNCM I-1422, une souche *S. thermophilus* CNCM I-1424, et une souche *L. delbruckii subsp. bulgaricus* CNCM I-1420. Chacune de ces souches sont utilisées traditionnellement dans la fabrication industrielle de yogourt, et ont été déposées, selon le traité de Budapest, le 18 mai 1994, à la Collection Nationale de Cultures de Microorganismes (CNCM), Institut Pasteur, 28 rue du Dr Roux, 75724 Paris Cedex 15, France. Les starters comprenant les souches CNCM I-1422 et CNCM I-1424 ont été préparés comme décrit à l'exemple 1, et le starter comprenant la souche CNCM I-1420 a été préparé comme décrit à l'exemple 2.

On ajoute donc au lait, 7 ml des starters congelés comprenant les souches CNCM I-1422 et CNCM I-1424, 6 ml du starter congelé comprenant la souche CNCM I-1420, on incube le lait à 42°C jusqu'à un pH d'environ 4,65, puis on le refroidit jusqu'à 4°C.

De même, on réalise une autre fermentation du lait entier pasteurisé avec un autre cocktail de trois starters congelés comprenant chacun respectivement une souche *S. thermophilus* CNCM I-1421, une souche *S. thermophilus* CNCM I-1423, et une souche *L. delbruckii subsp. bulgaricus* CNCM I-800. Chacune de ces souches sont utilisées traditionnellement dans la fabrication industrielle de yogourt, et ont été déposées, le 18.05.94 pour les souches CNCM I-1421 et I-1423, et le 4.10.88 pour la souche CNCM I-800, selon le traité de Budapest, à la Collection Nationale de Cultures de Microorganismes (CNCM), Institut Pasteur, 28 rue du Dr Roux, 75724 Paris Cedex 15, France. Les starters comprenant les souches CNCM I-1421 et CNCM I-1423 ont été préparés comme décrit à l'exemple 1, et le starter comprenant la souche CNCM I-800 a été préparé comme décrit à l'exemple 2.

On ajoute ainsi au lait 7 ml des starters congelés comprenant les souches CNCM I-1421 et CNCM I-1423, et 6 ml du starter congelé comprenant la souche CNCM I-800, on incube le lait à 42°C jusqu'à un pH d'environ 4,65, puis on le refroidit jusqu'à 4°C.

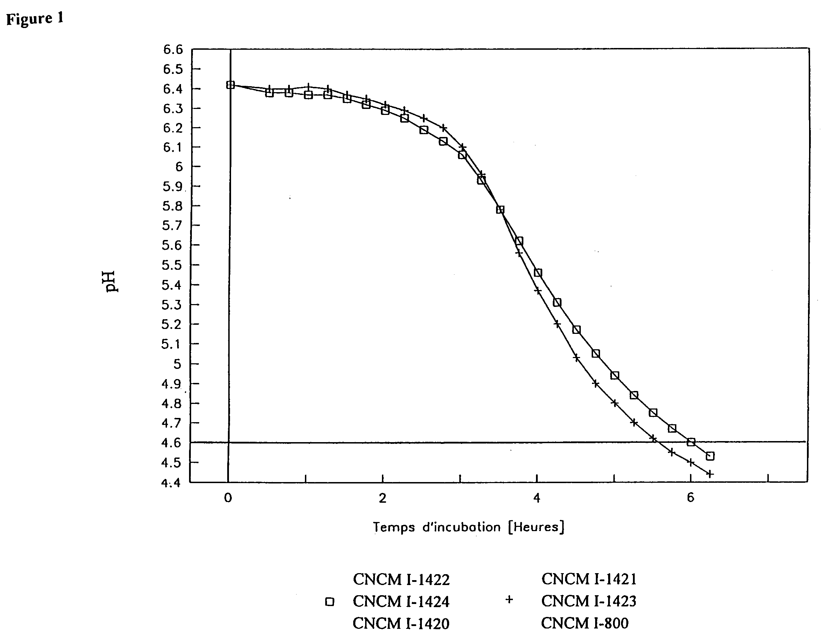

Les courbes d'acidification sont données sur la figure 1, et le tableau suivant illustre les caractéristiques des produits obtenus.

| Coktail de starters (CNCM) | Temps d'acidification jusqu'à pH 4,65 | pH du produit au bout d'1 jour (à 4°C) | pH du produit au bout de 24 jours (à 4°C) | Dégustation à 24 jours |
|---|---|---|---|---|
| I-1420 I-1422 I-1424 | 6 h | 4,34 | 4,1 | Bon goût Un peu acide Texture cassante |
| I-800 I-1421 I-1423 | 5 h 40 min | 4,35 | 4,2 | Bon goût Texture lisse |

**Exemple 5**

On prépare des yogourts brassés par inoculation directe avec des starters congelés tels que décrit dans les exemples 1 et 2.

Le lait est préparé à base de lait entier comprenant 3,7% de matière grasse et 2,5% de poudre de lait écrémé. On pasteurise 40 l de ce lait à 105°C pendant 2 min, on l'homogénéise ensuite à 75°C et 300 bar (premier étage), enfin on le refroidit jusqu'à une température d'environ 43°C.

On réalise alors une fermentation de ce lait avec un cocktail de trois starters congelés comprenant respectivement la souche CNCM I-1422, la souche CNCM I-1424 et la souche CNCM I-800. Pour ce faire, on ajoute au lait 7 ml des starters congelés décrits à l'exemple 4 comprenant les souches CNCM I-1422 et CNCM I-1424, 6 ml du starter congelé décrit à l'exemple 4 comprenant la souche CNCM I-800. On incube ensuite ce lait à 43°C jusqu'à un pH d'environ 4,65, puis on le refroidit jusqu'à 4°C tout en le brassant.

De même, on réalise une autre fermentation du lait entier avec un autre cocktail de trois starters congelés comprenant respectivement la souche CNCM I-1421, la souche CNCM I-1423, et la souche CNCM I-1420. Pour ce faire, on ajoute également au lait 7 ml des starters congelés décrits à l'exemple 4 comprenant les souches CNCM I-1421 et CNCM I-1423, 6 ml du starter congelé décrit à l'exemple 4 comprenant la souche CNCM I-1420. On incube ensuite ce lait à 43°C jusqu'à un pH d'environ 4,65, puis on le refroidit jusqu'à 4°C.

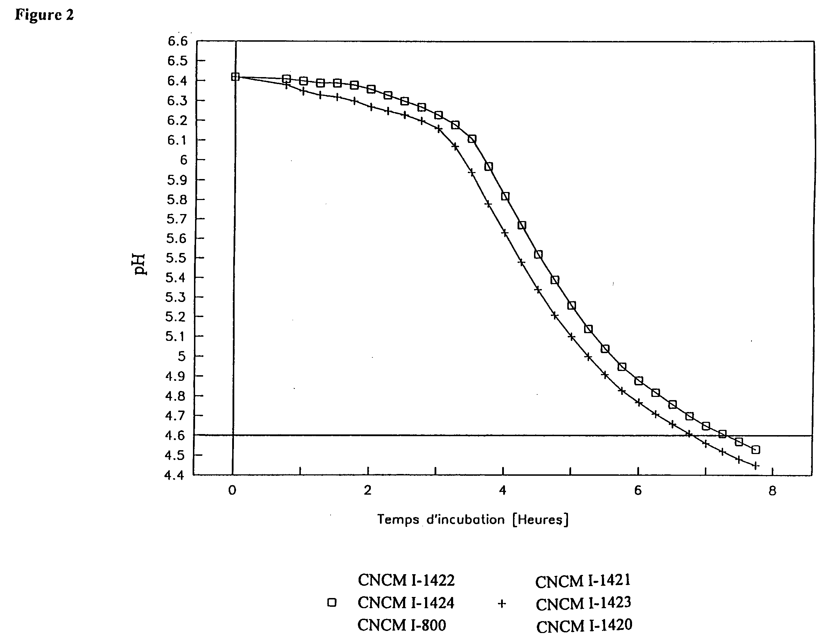

Les courbes d'acidification sont données sur la figure 2, et le tableau suivant illustre les caractéristiques des produits obtenus.

| Coktail de starters (CNCM) | Temps d'acidification jusqu'à pH 4,65 | pH du produit au bout d'1 jour (à 4°C) | pH du produit au bout de 24 jours (à 4°C) | Dégustation à 24 jours |
|---|---|---|---|---|
| I-1422 I-1424 I-800 | 7 h 15 min | 4,49 | 4,3 | Très bon goût Aromatique Texture lisse et onctueuse |
| I-1420 I-1421 I-1423 | 6 h 45 min | 4,55 | 4,32 | Bon goût Aromatique Texture onctueuse |

**Exemple 6**

On inocule le lait pasteurisé de l'exemple 4, avec 0,05% du coktail de starters congelés de l'exemple 5 comprenant, dans les mêmes proportions, les souches CNCM I-1422, I-1424 et I-800, puis on incube ce lait à 42°C jusqu'à pH 4,65.

Pour comparaison, on inocule ce même lait, avec 3% d'une culture fraîche traditionnelle comprenant, dans les mêmes proportions, les mêmes souches *S. thermophilus* et *L. delbruckii subsp. bulgaricus*, puis on incube à 42°C jusqu'à pH 4,65.

Pour comparaison, on inocule ce même lait avec 0,02% d'un starter congelé concentré traditionnel comprenant, dans les mêmes proportions, les mêmes souches *S. thermophilus* et *L. delbruckii subsp. bulgaricus*, puis on incube à 42°C jusqu'à pH 4,65.

Le tableau ci-après présente les résultats obtenus pour les trois types de fermentations.

| Caractéristiques des cultures | Culture fraîche | Coktail de starters congelés | Coktail traditionnel de starters concentrés et congelés |
|---|---|---|---|
| Nombre de cellules vivantes dans les starters (CFU/g) | $3.10^8$ | $2,5.10^8$ | $10^{10}$ |
| Inoculation directe (%) | 3 | 0,05 | 0,02 |
| Nombre de cellules vivantes dans le lait après inoculation (CFU/g) | $9.10^6$ | $1,3.10^5$ | $2.10^6$ |
| Temps de latence observé | 10 min | 1h | 1h |
| Temps prévu jusqu'à pH 4,65 (environ $5,3.10^8$ CFU/g) pour 100% d'activité des cellules | 3 h 7min | 7 h | 5 h |
| Temps réel jusqu'à pH 4,65 | 3 h 10min | 7h 5min | 6h 55min |
| Taux d'activité des cellules (T) | ( 100 % ) | > 95 % | < 10 % |

En admettant que le temps moyen de génération des cellules est de 30 min et que le taux d'activité des cellules issues de la culture fraîches est par référence de 100%, on peut déterminer approximativement le taux relatif d'activité fermentaire des cellules issues des starters congelés, par la relation qui suit.

$$T = 109 \times (5,3.10^8/(1,02338)^{(\text{temps réel} - \text{temps de latence en min})}/(\text{CFU/g dans le lait à l'inoculation})$$

On constate ainsi que les deux types de coktail de starters congelés présentent un temps de latence identique, dû certainement à une adaptation des cellules à leur nouvel environnement.

Cependant, ces deux types de coktail de starters ne fermentent pas le lait de la même manière. En effet, le coktail de starters congelés traditionnels fermente le lait en 6 h 55 min, alors qu'il devrait le fermenter en 5h, si on se base sur un temps de génération moyen de 30 min. Par contre, le coktail de starters selon la présente invention, fermente le lait quasiment dans le temps prévu.

On constate ainsi que plus de 95% des bactéries lactiques issues du starter selon la présente invention fermentent le lait, alors que moins de 10% des bactéries lactiques issues du starter congelé traditionnel fermentent ce lait.

C'est pourquoi, en fonction du pourcentage initial d'inoculation, le starter objet de la présente invention peut fermenter un lait jusqu'à un pH traditionnel de yogourt, c'est à dire jusqu'à pH 4,4 à 4,8, aussi rapidement que peut le faire un starter congelé traditionnel (en inoculation directe). Pour cela, on inocule de préférence un lait avec 2,5 fois plus de starter selon la présente invention par rapport au starter congelé traditionnel.

Enfin, comme on peut le déduire du tableau ci-dessus, pour un pourcentage d'inoculation égal à celui du starter congelé traditionnel, le starter selon la présente invention fermente le lait un peu moins rapidement que peut le faire un starter congelé traditionnel. Cependant, la perte de temps est minime, de l'ordre 20 min, et elle est largement compensée par le gain du prix de revient du starter de la présente invention, et la facilité avec laquelle on peut le fabriquer.

**Revendications**

1. Procédé de préparation d'un starter congelé pour l'inoculation directe d'un lait dans le but d'en faire du yogourt, caractérisé en ce que l'on cultive des bactéries lactiques dans du lait à une température de 40-45°C jusqu'à un pH de 4,3-5,5, puis l'on congèle directement la culture.

2. Procédé selon la revendication 1, caractérisé en ce que l'on cultive au moins une souche de streptocoques et/ou au moins une souche de lactobacilles.

3. Procédé selon la revendication 2, caractérisé par le fait qu'on inocule le lait avec une ou plusieurs précultures fraîches comprenant chacune une ou plusieurs souches de bactéries lactiques pouvant être d'un genre ou d'une espèce différente.

4. Procédé selon la revendication 2, caractérisé en ce que l'on ajoute à une culture de Lactobacilles 3-10% de glycérine avant de la congeler.

5. Procédé selon la revendication 1, caractérisé en ce que le lait comprend en outre 1% d'extrait de levure.

6. Procédé selon la revendication 1, caractérisé en ce que l'on congèle la culture entre -20 et -90°C.

7. Procédé selon l'une des revendications 1 à 6, caractérisé par le fait qu'on cultive des streptocoques à une température de 41-44°C jusqu'à un pH de 4,9-5,2 de manière à obtenir une concentration de cellules de l'ordre de $2.10^8$ à $5.10^8$ CFU/g, puis on congèle directement la culture.

8. Procédé selon l'une des revendications 1 à 6, caractérisé par le fait qu'on cultive des lactobacilles à une température de 41-44°C jusqu'à un pH de 4,6-5 de manière à obtenir une concentration de cellules de l'ordre de $10^8$ à $5.10^8$ CFU/g, puis on congèle directement la culture.

9. Procédé selon l'une des revendications 1 à 6, caractérisé par le fait qu'on cultive des lactobacilles et des streptocoques à une température de 41-44°C jusqu'à un pH de 4,5-4,9 de manière à obtenir une concentration de cellules de l'ordre de $3.10^8$ à $5.10^8$ CFU/g, puis on congèle directement la culture.

10. Procédé selon la revendication 9, caractérisé en ce que le mélange bactérien comprend 50-90% de streptocoques par rapport aux lactobacilles.

11. Starter congelé obtenu par le procédé selon l'une des revendications 1 à 10.

12. Utilisation du starter congelé selon la revendication 11 pour la fabrication de yogourt.

13. Utilisation selon la revendication 12 à raison de 0,01 à 0,1% dans du lait.

Figure 1

pH

Temps d'incubation [Heures]

|  | CNCM I-1422 |  | CNCM I-1421 |
|---|---|---|---|
| □ | CNCM I-1424 | + | CNCM I-1423 |
|  | CNCM I-1420 |  | CNCM I-800 |

**Figure 2**

CNCM I-1422      CNCM I-1421
CNCM I-1424  +  CNCM I-1423
CNCM I-800      CNCM I-1420

pH

Temps d'incubation [Heures]

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | US-A-3 897 307 (RANDOLF S. PORUBCAN) <br> * colonne 1, ligne 1 - ligne 55 * <br> * colonne 2, ligne 5 - ligne 20 * <br> * colonne 3; exemple A * <br> --- | 1-13 | C12N1/04 <br> A23C9/123 |
| X | CHEIRON, <br> vol.2, no.1, 1973, MADRAS <br> pages 52 - 54 <br> T. JAYACHANDRAN ET AL 'The effect of lyophylisation on the viability of certain strains of starter bacteria' <br> * le document en entier * <br> --- | 1-13 | |
| A | LE LAIT, <br> vol.51, 1971, PARIS <br> pages 437 - 453 <br> JABARIT, A. 'Contribution à l'étude comparative concernant le comportement physiologique et le taux de survie de cinq cultures mixtes dans le milieux cryoconcentrés et dans ceux enrichis par des substances protectrices et facteurs de croissances' <br> * le document en entier * <br> --- | 1-13 | |
| A | JEREMIJA LJ. RASIC ET AL 'Yoghurt' <br> 1987 , TECHNICAL DAIRY PUBLISHING HOUSE , COPENHAGEN <br> * page 186 - page 221 * <br> --- | 1-13 | DOMAINES TECHNIQUES RECHERCHES (Int.Cl.6) <br> C12N <br> A23C |
| A | STANLEY E. GILLILAND 'Bacterial starter cultures for foods' <br> 1985 , CRC PRESS INC , FLORIDA <br> * page 41 - page 55 * <br> * page 145 - page 157 * <br> ----- | 1-13 | |

Le présent rapport a été établi pour toutes les revendications

| Lieu de la recherche | Date d'achèvement de la recherche | Examinateur |
|---|---|---|
| LA HAYE | 10 Octobre 1994 | Fernandez y Branas,F |